Europäisches Patentamt

European Patent Office

Office européen des brevets

(11) Veröffentlichungsnummer : **0 495 757 A1**

## (12) EUROPÄISCHE PATENTANMELDUNG

(21) Anmeldenummer : **92810019.7**

(22) Anmeldetag : **14.01.92**

(51) Int. Cl.⁵ : **A61H 39/00**

(30) Priorität : **14.01.91 DE 9100366 U**

(43) Veröffentlichungstag der Anmeldung :
**22.07.92 Patentblatt 92/30**

(84) Benannte Vertragsstaaten :
**AT CH ES FR GB IT LI NL**

(71) Anmelder : **Jenoptik Jena G.m.b.H.
Carl-Zeiss-Strasse 1
O-6900 Jena (DE)**

(72) Erfinder : **Donnerhacke, Klaus, Dr.
Alte Strasse 5
O-6900 Jena (DE)**

(72) Erfinder : **Apitz, Jörg
Carolinenstrasse 28
O-6902 Jena (DE)**
Erfinder : **Börner, Klaus
Comeniusstrasse 98
O-8019 Dresden (DE)**
Erfinder : **Heinrich, Jörg
Hugo-Schrade-Str. 40
O-6908 Jena (DE)**
Erfinder : **Kutschki, Uwe
Dr. Salvador-Allende-Platz 21
O-6902 Jena (DE)**

(74) Vertreter : **Tschudi, Lorenz et al
Bovard AG Patentanwälte VSP
Optingenstrasse 16
CH-3000 Bern 25 (CH)**

(54) **Behandlungsstift zur Stimulation von Akupunkturpunkten.**

(57) Der Behandlungsstift zur Stimulation von Akupunkturpunkten ist in der Humanmedizin zur Akupunktur am menschlichen Lebewesen einsetzbar. Unter Nutzung einer Steuergerätekonzeption realisiert er insbesondere die drei Funktionen : Akupunkturpunktsuche, Elektro- und Laserakupunktur. Der Stift besteht im wesentlichen aus einer Behandlungsspitze (2), zwei Leiterplatten (6, 7) und drei Schaltelementen (4, 5, 9). Zwei Suchhilfen sind bei der Akupunkturpunktsuche vorhanden. Der Behandlungsspitze sind funktionell eine Kanüle (14) mit innenwandigem Lichtwellenleiter (15), eine Lichtquelle (16), ein Kühlkörper (18) und ein Magnet (21) zugeordnet. Die Leiterplatten besitzen je eine elektronische Schaltung zur Auswertung und Anzeige der Akupunkturpunktsuchergebnisse bzw. zur Ansteuerung der Lichtquelle. Das die Elemente des Stiftes ummantelnde Gehäuse (1) setzt sich aus einem Kreiszylinder- und einem Kreiskegelstumpfteil zusammen, die mit ihren Mantelflächen nahtlos ineinander übergehen. Der Magnet ist an einem Kühlkörper befestigt, der sich beim Eindrücken einer federnd gelagerten Behandlungsspitze unter ein magnetempfindliches Schaltelement bewegt. Eine Sicherheitsschaltung ist mit dem magnetempfindlichen Schaltelement verbunden, wobei nach dessen Aktivierung eine Freigabe der am Steuergerät vorgewählten Behandlungsart vorgesehen ist.

EP 0 495 757 A1

Fig. 2

zu Leiterplatte 7

Die Erfindung betrifft einen Behandlungsstift zur Stimulation von Akupunkturpunkten am menschlichen Lebewesen nach dem Oberbegriff des Patentanspruchs 1, der besonders bei Therapieformen, die nicht die klassische Nadel-Akupunktur anwenden, unter Nutzung einer Steuergerätekonzeption in der Humanmedizin dient.

Die Akupunktur (lat. acus = Nadel; punctura = Stich) ist eine mehrere tausend Jahre alte chinesische Heilmethode, die weltweit sehr verbreitet zu therapeutischen Behandlungen genutzt wird. Bei der klassischen Nadel-Akupunktur werden dünne Metallnadeln an bestimmten Hautstellen des menschlichen Körpers - den sogenannten Akupunkturpunkten - eingestochen. Der damit verbundene Reiz wird zur Stimulierung genutzt.

Eine vergleichbare therapeutische Wirkung läßt sich beispielsweise jedoch auch durch Einwirkung von elektrischer Energie (Elektropunktur) bzw. Strahlungsenergie (Laserpunktur) erzielen. Diese alternativen Methoden bieten eine Reihe von humanitären Vorteilen. Sie werden deshalb im zunehmenden Maße eingesetzt.

In den meisten bekannten Anordnungen zur Stimulation der Akupunkturpunkte und zur Akupunkturpunktsuche sind diese Gerätefunktionen in getrennten Handhabevorrichtungen realisiert. So ist beispielsweise eine Gerätelösung der Medical Electronics GmbH aus Lichtenau in der BRD bekannt, bei der mit einem Grundgerät LSI 2030 der Anschluß für ein Punktsuchgerät und für einen Laserapplikator separat ausgeführt ist. Ein Gerät der Firma ISKRA aus Ljubljana in Jugoslawien bietet mit einem Laser-Stimulator LS A-02 die getrennte Realisierung der einzelnen therapeutischen Funktionen mittels Elektro- oder Laserpunktur an, wobei die Suche der Akupunkturpunkte gesondert erfolgt. Die Berührung eines Knopfes am Gesamtgerät oder das unhandliche Drehen eines Kontaktringes an einem Behandlungsgriffel sichert hier die Laserbedienung, wobei am Gesamtgerät ein separater Anschluß für ein Elektrodenpaar vorhanden ist. Die Pitterling Electronic GmbH aus München in der BRD bietet weitestgehend vergegenständlichte Gerätelösungen zur Akupunkturpunktsuche oder zur Elektro-Akupunktur an, wobei getrennte Geräte zur Realisierung der unterschiedlichen Gebrauchsformen erforderlich sind. Eine Griffelumschaltung von Diagnose auf Therapie ist teilweise über verschiedene Gerätetypen realisiert. Die Ungarischen Optischen Werke in Budapest vertreiben einen tragbaren Soft-Laser für Akupunktur und Kosmetik des Types LE ZI, dessen Behandlungskopf neben dem Suchen des Akupunkturpunktes auch zum Starten einer Laserbestrahlung geeignet ist. Der Behandlungskopf stellt einen Punksuchgriffel mit eingebauter Faseroptik dar, der den elektrischen Widerstand der Hautoberfläche eines menschlichen Körpers detektiert, wobei proportional zu dessen relativer Änderung eine optische bzw. akustische Anzeige erfolgt. Nach dem Auffinden des gesuchten Akupunkturpunktes kann eine Umschaltung durch Betätigung eines Schalters mittels Radbewegung am Griffel auf die Laserbestrahlung erfolgen. Eine Lichtsperre als Sicherheitseinrichtung für ungewollte Bestrahlung wird über eine mechanische Konstruktion mit Federkontakt (über eine Sonde) betätigt, in dem die erwähnte Rädchenbedienung am Griffel durch entspannten Federkontakt das Schließen eines elektrischen Schaltkontaktes sichert. Hierdurch soll die Lichtsperre aufgehoben sein.

Die separate Handhabung der getrennten Akupunkturfunktionen, insbesondere die Trennung der Akupunkturpunktsuche von den therapeutischen Behandlungsmethoden, bewirkt nachteiligerweise, daß ein über Widerstandmessung aufgefundener Akupunkturpunkt, beispielsweise in der Größe von etwa 1mm Durchmesser auf der Hautoberfläche, durch den erforderlichen Wechsel des Behandlungsstückes nur ungenauere Erfolgschancen zum genaueren Wiedertreffen bietet. Eine weitere wichtige Voraussetzung zur erfolgreichen Stimulation mit Lichtstrahlung ist aber die genaue Plazierung der punktförmig aufzubringenden Lichtstrahlung; im Unterschied zur klassischen Nadelstimulation oder Elektrotherapie, bei der eine gewisse Umgebungsbeeinflussung bei in Grenzen gehaltener Ungenauigkeit der Plazierung noch eine erfolgreiche Behandlung ermöglicht. Bei der Suche der Akupunkturpunkte, die sich durch einen niedrigeren Widerstand als die sie umgebenhen Hautareale auszeichnen, ist außerdem die Tatsache zu beachten, daß der Hautwiderstand druckabhängig gemessen wird, wobei ein zu hoher Aufsetzdruck der Meßspitze den elektrischen Wirderstand der Haut an der Druckstelle erniedrigt. Es besteht die Gefahr, daß sich hierbei Meßfehler entwickeln bzw. sich eine falsche Akupunkturpunktbestimmung abzeichnet, die von allen bekannten Lösungen problemorientiert und zu ihrem Nachteil nicht abgestellt sind.

Bekannte Lösungen der Patentliteratur bestätigen den bisher geschilderten Sachverhalt. Es sind weitestgehend auch hier Lösungen offenbart, die mit den bereits erwähnten Nachteilen unterschiedliche Gerätekonzepte mit den einzelnen Gerätefunktionen in verschiedenen Handhabevorrichtungen realisieren. Beispielgebend seien die Lösungen oder Erfindungsbeschreibungen: DE 36 31 600, DE 37 21 271, EP 0099662, EP 0160753 für die Elektrostimulation;DE 32 37 398, DE 2 619 930, DE 2 620 846, DE 84 10 442 für die Lasertherapie genannt. Lösungeng zur Bestimmung der Akupunkturpunkte sind in den DE 24 31 183, DE 28 10 344, DE 36 38 003 beispielsweise offenbart. Weiterhin sind ebenso Lösungen veröffentlicht, die neben der Akupunkturpunktsuche auch die Elektrotherapie am Patienten mit einem Grundgerät, aber mit verschiedenen Behandlungsstiften oder mit einer unhandlichen Umschaltungsmöglichkeit an Stift selbst ermöglichen. Beispielgebend für derartige Lösungen seien die Erfindungsbeschreibungen; DD 221 082, DE 36 12 646 genannt. Alle diese bekannten technischen Lösungen bieten kein Gerätekonzept zum universellen Gebrauch mehrerer Akupunkturmöglichkeiten einschließlich der voranzustellenden Akupunkturpunktsuche an.

Mit ihnen ist entweder nur eine getrennte Diagnose und Therapie in der Akupunktur möglich oder der erforderliche Wechsel verschiedener Behandlungsgriffel, die nur bestimmte Akupunkturfunktionen gerätetechnisch angepaßt umsetzen, notwendig. Die unhandliche Handhabung der Griffel, einbezogen das Behandeln der gefundenen Akupunkturpunkte eines Patienten nach einem Wechsel der Handhabetechnik, führt zur Einschränkung des möglicherweise erwarteten Behandlungserfolges durch den Therapeuten. So ist die Akupunktur mit Laserstrahlen nur bei deren genau fixierten Richtung auf den entsprechenden Akupunkturpunkt erfolgreich. Die genaue Plazierung der punktförmig aufzubringenden Lichtstrahlung auf einen über Widerstandsmessung aufgefundenen Akupunkturpunkt von < 1 mm Durchmesser auf der Hautoberfläche ist deshalb von großer Wichtigkeit. Sie wird durch den Wechsel der Behandlungsgriffel mit allen im Stand der Technik bekannten Vorrichtungen einbezogen deren Handhabung nicht garantiert, wobei auch die genannten Ungenauigkeiten der Plazierung nur bei Einhaltung von Toleranzgrenzen eine erfolgreiche Elektro-Akupunktur ermöglichen.

Aufgabe der Erfindung ist es, einen Behandlungsstift zur Stimulation von Akupunkturpunkten zu schaffen, in dem in Verbindung mit einem Steuergerät die Funktion der genauen Lagebestimmung eines Akupunkturpunktes am menschlichen Körper und die therapeutischen Behandlungsmethoden der Stimulation der zu behandelnden Akupunkturpunkte wahlweise durch Lichtstrahlung oder elektrischen Strom vereint sind. Er ist so zu optimieren, daß es ohne sein Absetzen vom Körper des Patienten möglich ist, sofort nach Auffinden des Akupunkturpunktes eine Licht- oder Elektrostimulation durchzuführen.

Diese Aufgabe für einen Behandlungsstift zur Stimulation von Akupunkturpunkten, der über eine Leitung mit einem Steuergerät verbunden ist, wobei ein separater Masseanschluß aus dem Steuergerät geführt ist, der mit einem Gehäuse ummantelt ist, das sich aus einem Kreiszylinder und einem Kreiskegelstumpf formorientiert zusammensetzt, wobei diese beiden Gehäuseteile an ihrer Grundfläche mit ihren Mantelflächen nahtlos ineinander übergehen und eine Deckfläche in Form einer Kugelkalotte das Kreiszylinderteil nahtlos zudeckt und die Deckfläche des Kreiskegelstumpfteiles zur paßgerechten Einführung einer Führugsbuchse vorgesehen ist, der auf seinem Gehäuse an einer auf dem Kreiszylinderteil aufgesetzten ebenen Fläche eine optische Anzeige besitzt, die vorzugsweise mit einem LED-Leuchtband realisiert ist, bei dem außerhalb des Gehäuse eine Wippe angeordnet ist, die eine geeignete Handhabung im Umgang mit dem Gerät ermöglicht und wahlweise zwei Schaltelemente betätigt, bei dem eine Leiterplatte mit einer elektronischen Schaltung zur Auswertung und Anzeige der Akupunkturpunktsuchergebnisse angeordnet und auf die optische Anzeige geschaltet ist, bei dem im weitestgehend vorderen kegelförmigen Teil des Gehäuseinneren eine Behandlungsspitze angeordnet ist, zu der im wesentlichen eine Kanüle mit leitfähiger Ummantelung gehört und an der eine weitere Leiterplatte mit einer elektronischen Schaltung zur Ansteuerung einer Lichtquelle befestigt ist, bei dem die Leiterplatte zur elektronischen Auswertung und Anzeige der Akupunkturpunktsuchergebnisse mit der weiteren Leiterplatte zur elektronischen Ansteuerung der Lichtquelle verbunden ist, wobei die weitere Leiterplatte als flexible Leiterplatte ausgeführt ist, bei dem eine leitfähige Verbindung zwischen dieser weiteren Leiterplatte und der Kanüle vorgesehen ist, bei dem die genannte Führungsbuchse an einem Gestell befestigt ist, wobei diese Führungsbuchse vorzugsweise in eine Aussparung des Gestells eingepaßt ist, bei dem die Kanüle in dieser Führungsbuchse längsverschieblich gelagert und mit eine Fassung, die zur Aufnahme der Lichtquelle vorgesehen ist, bei dem die Kanüle mit einer elektronischen Schaltung zur Sicherung der wahlweisen Stromzufuhr für eine Akupunkturpunktsuche und eine Elektrostimulation verbunden ist, bei dem in der Kanüle innenwandig ein Lichtwellenleiter angeordnet ist, in dem eine von der Lichtquelle initiierte Lichtstrahlung direkt einkoppelbar ist und deren Auskopplung am anderen Ende des Lichtwellenleiters für eine Lichtstimulation vorgesehen ist bei dem alle Verbindungen elektrisch leitend gestaltet sind, wird durch die im kennzeichnenden Teil der Patentansprüche angegebenen Merkmale gelöst. Ein Magnet, der sich beim Eindrücken einer federnd gelagerten Behandlungsspitze unter ein magnetempfindliches Schaltelement bewegt, ist an einem Kühlkörper befestigt. Eine Sicherheitsschaltung ist mit dem magnetempfindlichen Schaltelement verbunden, wobei nach erfolgter Aktivierung dieses Schaltelementes eine Freigabe der am Steuergerät vorgewählten Behandlungsart vorgesehen ist. Die von der Wippe betätigten Schaltelemente sind gleichzeitig zur Einstellung des Behandlungsstromes bei der Elektrostimulation vorgesehen.

Als Schaltelemente sind im Behandlungsstift vorzugsweise Schalter eingesetzt. Die von der Wippe betätigten Schaltelemente sind unmittelbar unterhalb derselben im Inneren des Gehäuses angeordnet. Das magnetempfindliche Schaltelement ist ein elektronischer Schalter, vorzugsweise ein Hall-Schaltkreis.

Es ist eine leitungsmäßige Verknüpfung von einem durch die Wippe betätigten Schaltelement und von dem magnetempfindlichen Schaltelement zur Sicherheitsschaltung vorgesehen, wodurch mit dieser leitungsmäßigen Verknüpfung eine Behandlung nur bei gleichzeitiger Betätigung des erstgenannten Schaltelementes durch ein Eindrücken der federnd gelagerten Behandlungsspitze beim Aufsetzen derselben, vorzugsweise auf den Behandlungsort eines Patienten, ermöglicht ist.

Die Lichtquelle ist im Behandlungsstift ein in ihm angeordneter Halbleiterlaser, der innerhalb der Fassung lokalisiert ist. Der Einsatz einer Laserdiode als Halbleiterlaser ist möglich. Das Gehäuse des Behandlungsstiftes besteht aus einem geeigneten Plastmaterial oder einem plastbeschichteten Material, das den Ansprüchen der elektrischen Durchschlagsfestigkeit genügt. Der eingesetzte Magnet im Stift ist als Elektro- oder Dauermagnet ausgeführt.

Die Kanüle besteht aus einem nichtrostenden und zur Sterilisation geeigneten Metall oder Edelmetall oder Plastwerkstoff mit leitfähigem Metall- oder Edelmetallüberzug. Sie ist gegenüber dem Gestell und gegenüber der Fassung elektrisch isoliert.

Die Behandlungsspitze ist durch eine geeignete und ausreichend dimensionierte Zugfeder federnd gelagert.

Mit der Kanüle ist die Auskopplung eines Lichtstrahlenbündels aus dem anderen Ende des Lichtwellenleiters direkt und damit einem Durchmesser < 1 mm, vorzugsweise $\leqq$ 0,5 mm, vorgesehen, Außerhalb des Gehäuses mündet die Kanüle in Richtung des Behandlungsortes in eine kalottenförmige Kontaktfläche. Der in der Kanüle unbeweglich geführte Lichtwellenleiter ist am Ende der kalottenförmigen Kontaktfläche plan abgeschlossen.

Parallel zur optischen Anzeige am Stift ist im Stift eine akustische Suchhilfe integriert, die wahlweise abschaltbar ist und die Akupunkturpunktsuche akustisch unterstützt.

Die durch die Erfindung erreichten Vorteile sind im wesentlichen darin zu sehen, daß es ohne Wechsel bzw. Absetzen des Behandlungsstiftes schnell und sicher möglich ist, sofort nach Auffinden des Akupunkturpunktes eine Laser- oder Elektrostimulation durchzuführen. Bemerkenswert ist neben der hohen Sicherheit bei der Lokalisierung der Akupunkturpunkte die einfache und problemlose Handhabung des Behandlungsstiftes. Durch eine Sicherheitsschaltung wird die Laserstrahlung erst dann freigegeben, wenn der Behandlungsstift aufgesetzt ist. Damit wird bei der Laserpunktur eine Außengefährdung ausgeschlossen. Sie überwacht auch den zuverlässigen Spitzenwert des Therapiestromes, wobei bei einer Überschreitung dieses Stromwertes derselbe automatisch abgeschaltet und von einem nachgeschalteten Steuergerät als Fehler angezeigt wird.

Die unklompizierte Bedienung ermöglicht einem mit den Grundlagen der Akupunktur vertrauten Therapeuten die sofortige Benutzung der erfindungsgemäßen Lösung.

Weitere Vorteile der Erfindung gehen aus den Ansprüchen 2 bis 10 hervor.

So bietet die Ausgestaltung nach Anspruch 2 die Gewähr, daß die Lichtquelle erst betrieben werden kann, wenn der Behandlungsstift aufgesetzt ist.

Die Handhabung des Stiftes durch eine federnd gelagerte Behandlungsspitze nach Anspruch 6 ermöglicht dem Therapeuten, daß er mit seiner den Behandlungsstift führenden Hand einen annähernd konstanten Aufsetzpunkt der auf der Hand des Patienten sitzenden Kanüle über einen gewissen Bewegungsbereich realisiert; der bei der Akupunkturpunktsuche in Verbindung mit der vorteilhaften Ausgestaltung der Kanüle nach Anspruch 9 reale elektrische Widerstandsmeßwerte der Haut zur Bewertung der zu lokalisierenden Akupunkturpunkte des Patienten gewährt.

Die genaue Plazierung der Lichtstrahlung auf den ausgemessenen Akupunkturpunkt mit einem möglichst konstanten Druck ist durch eine Ausgestaltung der Kanüle nach Anspruch 7 möglich. Ebenso genau ist die Zufuhr des elektrischen Stromes auf diesen Punkt gerichtet, die durch die vorteilhafte Ausführung der Kanüle mit einem nichtrostenden und zur Sterilisation geeigneten Metall oder Edelmetall oder Plastwerkstoff mit leitfähigen Metall- oder Edelmetallüberzug die verlustarme und hygenische Zufuhr des Therapiestromes garantiert.

Eine vorteilhafte Ausgestaltung nach Anspruch 4 mit dem Einsatz einer Laserdiode als Halbleiterlaser bzw. Lichtquelle ist der Sicherheit von Therapeut und Patient zur Vermeidung einer unzulässig stark gerichteten Lichtstrahlung vorbehalten. Die Ausgestaltung nach Anspruch 10 ist insofern vorteilhaft, da die akustische Suchhilfe dem behandelnden Therapeuten eine zusätzliche Unterstützung zur optischen Anzeige bei der Akupunkturpunktsuche gewährt, die ihm bei schwer zugänglichen Körperregionen die Punktsuche erleichtert.

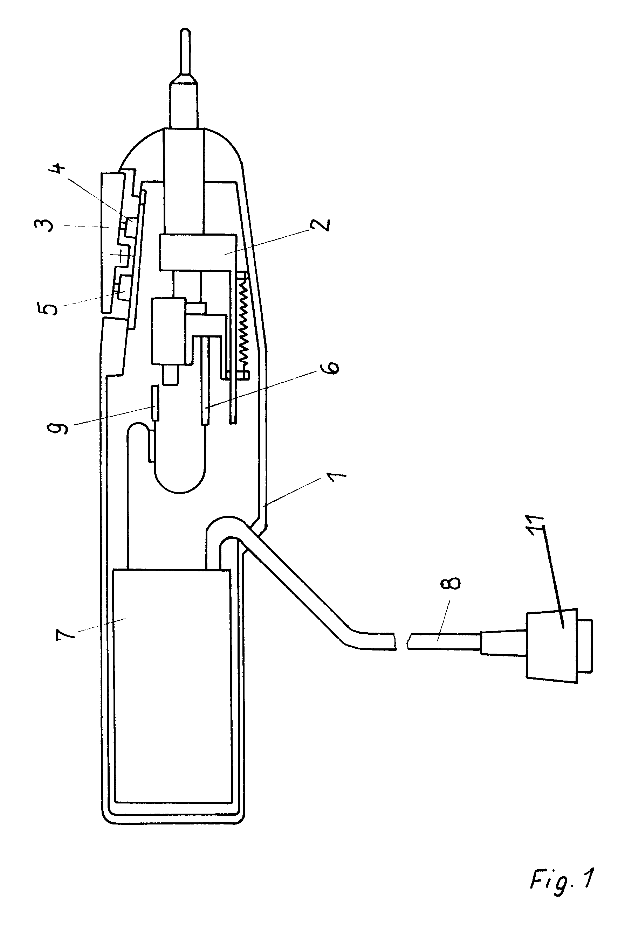

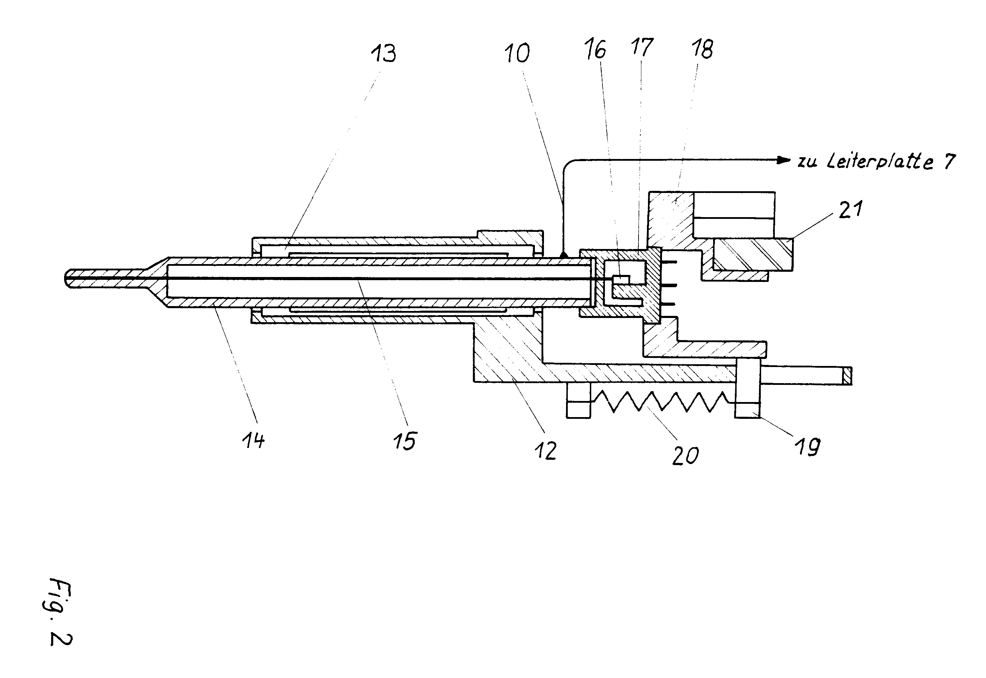

Ein Ausführungsbeispiel der Erfindung wird anhand der Figur 1 und 2 erläutert. Es zeigen:

Fig. 1 den Behandlungsstift zur Stimulation von Akupunkturpunkten;

Fig. 2 die Behandlungsspitze des Behandlungsstiftes zur Stimulation von Akupunkturpunkten

Der Behandlungsstift zur Stimulation von Akupunkturpunkten ist über eine Leitung 8 mittels Steckverbindung, die durch einen Gerätestecker 11 realisiert wird, mit einem Steuergerät verbunden. Aus dem Steuergerät, daß die Stromversorgung und Steuerung der verschiedenen Funktionen für die Behandlungsarten - Akupunkturpunktsuche - Licht- und Elektropunktur übernimmt, ist überdies ein separater Massekontakt geführt, der am Körper des zu behandelnden Patienten in geeigneter Art und Weise befestigt ist. Der Behandlungsstift selbst besitzt ein Gehäuse 1, das sich aus einem Kreiszylinder und einem Kreiskegelstumpf formorientiert zusammensetzt. Die beiden Gehäuseteile gehen an ihrer Grundfläche mit ihren Mantelflächen nahtlos ineinander über. Eine Deckfläche deckt das Kreiszylinderteil in Form einer Kugelkalotte nahtlos zu. Die Deckfläche des Kreiskegelstumpfteiles ist zur paßgerechten Einführung einer Führungsbuchse 13 vorgesehen. Das Gehäuse 1 besteht aus einem geeigneten Plastmaterial oder einem geeignetem plastbeschichtetem Material, das den Ansprüchen der elektrischen Durchschlagfestigkeit genügt.

In Fig. 1 ist der Aufbau des Behandlungsstiftes als eine mögliche Ausführungsform erkennbar. Er besteht aus dem erwähnten Gehäuse 1, auf dem an einer auf dem Kreiszylinderteil aufgesetzten ebenen Fläche eine optische Anzeige angeordnet ist. Diese Anzeige ist vorzugsweise mit einem LED-Leuchtband realisiert. Außerhalb des Gehäuses 1 ist eine Wippe 3 angeordnet. Sie betätigt wahlweise zwei Schaltelemente 4,5, die vorzugsweise als mechanisch betätigte Schalter integriert sind. Diese Schaltelemente 4,5 sind unmittelbar unterhalb der Wippe 3 im Inneren des Gehäuses 1 angeordnet. Eine Leiterplatte 7 mit einer elektronischen Schaltung zur Auswertung und Anzeige der Akupunkturpunktsuchergebnisse ist auf die erwähnte optische Anzeige leitungsmäßig geschalten. Im weitestgehend vorderen kegelförmigen Teil des Gehäuseinneren befindet sich eine Behandlungsspitze 2, zu der im wesentlichen eine in Fig. 2 dargestellte Kanüle 14 mit leitfähiger Ummantelung gehört. An dieser Kanüle 14 ist eine weitere Leiterplatte 6 mit einer elektronischen Schaltung zur Ansteuerung einer in Fig. 2 dargestellten Lichtquelle 16 befestigt. Die beiden Leiterplatten 6,7 sind leitungsmäßig miteinander verbunden, wobei die weitere Leiterplatte 6 als flexible (austauschbare) Leiterplatte ausgeführt ist.

In der Fig. 2 ist das Kernstück des Behandlungsstiftes die genannte Behandlungsspitze 2 detailliert dargestellt. Hier ist ersichtlich, daß eine leitfähige Verbindung 10 von der Kanüle 14 zur weiteren Leiterplatte 7 führt. Über diese Leiterplatte 7 ist die Kanüle 14 mit der Leiterplatte 6 zur Sicherung der wahlweisen Stromzufuhr für eine Akupunkturpunktsuche und eine Elektrostimulation verbunden. Die genannte Führungsbuchse 13 ist an einem Gestell 12 befestigt, wobei sie vorzugsweise in eine Aussparung des Gestells 12 eingepaßt ist. Die Kanüle 14 ist in dieser Führungsbuchse 13 längsverschieblich gelagert und mit einer Fassung 17, die zur Aufnahme der Lichtquelle vorgesehen ist, fest verbunden. Die Kanüle 14 besteht aus einem nichtrostenden und zur Sterilisation geeigneten Metall oder Edelmetall oder Plastwerkstoff mit leitfähigem Metall- oder Edelmetallüberzug. Sie ist gegenüber dem Gestell 12 und gegenüber der Fassung 17 elektrisch isoliert. Die innerhalb der Fassung 17 angeordnete Lichtquelle 16 ist ein Halbleiterlaser, für den vorteilhafterweise eine Laserdiode einsetzbar ist. Die Kanüle 14 mündet außerhalb des Gehäuses 1 in Richtung des Behandlungsortes in eine kalottenförmige Kontaktfläche ein. Innenwandig ist in ihr ein Lichtwellenleiter 15 angeordnet, in dem eine von der Lichtquelle 16 initiierte Lichtstrahlung direkt einkoppelbar ist. Der in der Kanüle 14 geführte Lichtwellenleiter 15 ist unbeweglich angeordnet. Am anderen Ende der kalottenförmigen Kontaktfläche schließt er plan mit dieser Kontaktfläche ab. Die Auskopplung dieser Lichtstrahlung geschieht am anderen Ende des Lichtwellenleiters 15. Die Auskopplung des für eine Lichtstimulation benötigten Lichtstrahlenbündels, wozu gebündelte Laserstrahlen, die von der möglicherweise installierten Laserdiode initiiert sind, bestens geeignet sind, am anderen Ende des Lichtwellenleiters 15 erfolgt mit der Kanüle 14 direkt und mit einem Durchmesser < 1 mm. Im Rahmen der beispielsgemäßen Realisierung ist ein Durchmesser von $\leqq 0{,}5$ mm angemessen. In der Behandlungsspitze 2 ist ein Magnet 21 an einem Kühlkörper 18 befestigt. Der Magnet 21, der sowohl als Elektromagnet aber auch als Dauermagnet realisiert ist, bewegt sich beim Eindrücken der Behandlungsspitze 2, die federnd gelargert ist, unter ein magnetempfindliches Schaltelement 9. Dieses Schaltelement, welches in Fig. 1 einsehbar ist, ist als elektronischer Schalter zu verstehen, wobei es beispielgemäß als Hall-Schaltkreis realisiert ist. Die Behandlungsspitze 2 ist durch eine geeignete Feder 20 federnd gelagert. Diese Feder 20 ist als ausreichend dimensionierte Zugfeder beispielsgemäß anzuordnen. Im weiteren ist dieses magnetempfindliche Schaltelement 9 mit einer Sicherheitsschaltung verbunden, wobei nach einer erfolgten Aktivierung des elektronischen Schalters eine Freigabe der am Steuergerät vorgewählten Behandlungsart erfolgt. Die von der Wippe 3 betätigten Schalter dienen anwendungsgerecht gleichzeitig zur Einstellung des Behandlungsstromes bei der Elektrostimulation des Patienten. Die leitungsgemäße Verknüpfung von einem durch die Wippe 3 betätigten Schaltelement 4 und vom magnetempfindlichen Schaltelement 9 zur Sicherheitsschaltung dient dazu, daß mit der Sicherheitsschaltung und bei gleichzeitiger Betätigung des Schaltelementes 4 und des magnetempfindlichen Schaltelementes 9 durch ein Eindrücken der federnd gelagerten Behandlungsspitze 2 auf den Behandlungsort ermöglicht ist.

Für den Gebrauch des Behandlungsstiftes zur Stimulation von Akupunkturpunkten am Patienten wird dieser mittels Gerätestecker 11 an das Steuergerät angeschlossen. Nach dem Einschalten des genannten Grundgerätes ist sofort die Funktion der Punktsuche aktiv. Zur Anpassung der Messung an die verschiedenen Hautareale setzt man den Stift auf die Haut des Patienten mit der Behandlungsspitze 2 auf und betätigt die Wippe 3 am hinteren Ende, wodurch der Schalter 5 geschlossen und die Eichung ausgeführt wird. Die Punktsuche erfolgt dann durch ein einfaches Überstreichen der Haut unter leichtem Druck, wobei der Hautwiderstand am LED-Leuchtband angezeigt wird. Ist der entsprechend gesuchte Akupunkturpunkt auf der Haut des Patienten gefunden, drückt der behandelnde Therapeut die Behandlungsspitze 2 weiter in den Behandlungsstift ein und betätigt danach die Wippe 3 am zur Kanüle 14 zeigenden vorderen Teil. So wird einerseits der magnetempfindliche Schalter 9 aktiviert sowie der Schalter 4 geschlossen, wodurch die Sicherheitsschaltung freigegeben und ausgelöst wird. Am Grundgerät erfolgt dabei schon vorher die Auswahl, ob die Behandlung als Laser- oder Elektrostimulation vorgenommen wird. Bei der Elektrostimulation dient der Schalter 4 gleichzeitig der Einstellung des Behandlungsstromes. Die Stimulation des Patienten mit elektrischem Strom geschieht über die Stromzufuhr im Leiter 10, wobei die leitfähig ummantelte Kanüle 14 den Stromtransport zur Akupunkturpunkt gewährleistet. Die beispielgemäß von der Laserdiode initiierte gebündelte Laserstrahlung wird direkt auf die Haut des Patienten ausgekoppelt.

Zu erwähnen ist, daß am Kühlkörper 18 ebenfalls die Laserdiodenfassung 17 befestigt ist. Die erwähnte Zugfeder 20 ist am Gestell 12 federnd über einen Stift 19 aufgehängt. Dadurch wird erst ein für die Behandlung benötigter und nahezu gleichmäßiger sowie geringfügiger Auflagedruck erreicht. Vorteilhafterweise unterstützt eine zusätzliche akustische Suchhilfe, beispielsweise ein Knack-Generator, der parallel zur optischen Anzeige angeordnet und wahlweise abschaltbar ist, die Akupunkturpunktsuche, deren Ergebnis die erfolgreiche therapeutische Behandlung der Patienten mittels Laser- oder Elektrostimulation erst gewährt.

## Patentansprüche

1. Behandlungsstift zur Stimulation von Akupunkturpunkten, der über eine Leitung (8) mit einem Steuergerät verbunden ist, wobei ein separator Masseanschluß aus dem Steuergerät geführt ist, der mit einem Gehäuse (1) ummantelt ist, das sich aus einem Kreiszylinder und einem Kreiskegelstumpf formorientiert zusammensetzt, wobei diese beiden Gehäuseteile an ihrer Grundfläche mit ihren Mantelflächen nahtlos ineinander übergehen und eine Deckfläche in Form einer Kugelkalotte das Kreiszylinderteil nahtlos zudeckt und die Deckfläche des Kreiskegelstumpfteiles zur paßgerechten Einführung einer Führungsbuchse (13) vorgesehen ist, der auf seinem Gehäuse (1) an einer auf dem Kreiszylinderteil aufgesetzten ebenen Fläche eine optische Anzeige besitzt, die vorzugsweise mit einem LED-Leuchtband realisiert ist, bei dem außenhalb des Gehäuses (1) eine Wippe (3) angeordnet ist, die eine geeignete Handhabung im Umgang mit dem Gerät ermöglicht und wahlweise zwei Schaltelemente (4,5) betätigt, bei dem eine Leiterplatte (7) mit einer elektronischen Schaltung zur Auswertung und Anzeige der Akupunkturpunktsuchergebnisse angeordnet und auf die optische Anzeige geschaltet ist, bei dem im weitestgehend vorderen kegelförmigen Teil des Gehäuseinneren eine Behandlungsspitze (2) angeordnet ist, zu der im wesentlichen eine Kanüle (14) mit leitfähiger Ummantelung gehört und an der eine weitere Leiterplatte (6) mit einer elektronischen Schaltung zur Ansteuerung einer Lichtquelle (16) befestigt ist, bei dem die Leiterplatte (7) zur elektronischen Auswertung und Anzeige der Akupunkturpunktsuchergebnisse mit der weiteren Leiterplatte (6) zur elektronischen Ansteuerung der Lichtquelle (16) verbunden ist, wobei die weitere Leiterplatte (6) als flexible Leiterplatte ausgeführt ist, bei dem eine leitfähige Verbindung (10) zwischen dieser weiteren Leiterplatte (7) und der Kanüle (14) vorgesehen ist, bei dem die genannte Führungsbuchse (13) an einem Gestell (12) befestigt ist, wobei diese Führungsbuchse (13) vorzugsweise in eine Aussparung des Gestells (12) eingepaßt ist, bei dem die Kanüle (14) in dieser Führungsbuchse (13) längsverschieblich gelagert und mit einer Fassung (17), die zur Aufnahme der Lichtquelle (16) vorgesehen ist, bei dem die Kanüle (14) mit einer elektronischen Schaltung zur Sicherung der wahlweisen Stromzufuhr für eine Akupunkturpunktsuche und eine Elektrostimulation verbunden ist, bei dem in der Kanüle (14) innenwandig ein Lichtwellenleiter (15) angeordnet ist, in dem eine von der Lichtquelle (16) initiierte Lichtstrahlung direkt einkoppelbar ist und deren Auskopplung am anderen Ende des Lichtwellenleiters (15) für eine Lichtstimulation vorgesehen ist, bei dem alle Verbindungen elektrisch leitend gestaltet sind, dadurch gekennzeichnet, daß ein Magnet (21) an einem Kühlkörper (18) befestigt ist, der sich beim Eindrücken einer federnd gelagerten Behandlungsspitze (2) unter ein magnetempfindliches Schaltelement bewegt, daß eine Sicherheitsschaltung mit dem magnetempfindlichen Schaltelement (9) verbunden ist, wobei nach erfolgter Aktivierung dieses Schaltelementes eine Freigabe der am Steuergerät vorgewählten Behandlungsart vorgesehen ist, daß die von der Wippe (3) betätigten Schaltelemente (4,5) gleichzeitig zur Einstellung des Behandlungsstromes bei der Elektrostimulation vorgesehen sind.

2. Behandlungsstift zur Stimulation von Akupunkturpunkten nach Anspruch 1, dadurch gekennzeichnet, daß eine leitungsmäßige Verknüpfung von einem durch die Wippe (3) betätigten Schaltelement (4) und von dem magnetempfindlichen Schaltelement (9) zur Sicherheitsschaltung vorgesehen ist, wodurch mit ihr nur bei gleichzeitiger Betätigung des erstgenannten Schaltelemente durch ein Drücken der Wippe (3) und des zweitgenannten Schaltelementes durch ein Eindrücken der federnd gelagerten Behandlungsspitze (2) beim Aufsetzen derselben, vorzugsweise auf den Behandlungsort eines Patienten, eine Behandlung ermöglicht ist.

3. Behandlungsstift zur Stimulation von Akupunkturpunkten nach Anspruch 1, dadurch gekennzeichnet, daß die Lichtquelle (16) ein Halbleiterlaser ist, der innerhalb der Fassung (17) angeordnet ist.

4. Behandlungsstift zur Stimulation von Akupunkturpunkten nach Anspruch 3, dadurch gekennzeichnet, daß als Halbleiterlaser eine Laserdiode eingesetzt ist.

5. Behandlungsstift zur Stimulation von Akupunkturpunkten nach Anspruch 1, dadurch gekennzeichnet, daß die Kanüle (14) gegenüber dem Gestell (12) und gegenüber der Fassung (17) elektrisch isoliert ist.

6. Behandlungsstift zur Stimulation von Akupunkturpunkten nach Anspruch 1, dadurch gekennzeichnet, daß die Behandlungsspitze (2) durch eine geeignete und ausreichend dimensionierte Zugfeder federnd gelagert ist.

7. Behandlungsstift zur Stimulation von Akupunkturpunkten nach Anspruch 1, dadurch gekennzeichnet, daß mit der Kanüle (14) die Auskopplung eines Lichtstrahlenbündels aus dem anderen Ende des Lichtwellenleiters (15) direkt und mit dem Durchmesser < 1mm, vorzugsweise von $\leqq$ 0,5 mm vorgesehen ist.

8. Behandlungsstift zur Stimulation von Akupunkturpunkten nach Anspruch 1, dadurch gekennzeichnet, daß die Kanüle (14) außerhalb des Gehäuses (1) in Richtung des Behandlungsortes in eine kalottenförmige Kontaktfläche mündet.

9. Behandlungsstift zur Stimulation von Akupunkturpunkten nach Anspruch 1 und Anspruch 8, dadurch gekennzeichnet,
daß der in der Kanüle (14) geführte Lichtwellenleiter (15) unbeweglich angeordnet und am Ende der kalottenförmigen Kontaktfläche plan abgeschlossen ist.

10. Behandlungsstift zur Stimulation von Akupunkturpunkten nach Anspruch 1, dadurch gekennzeichnet, daß parallel zur optischen Anzeige eine akustische Suchhilfe angeordnet ist, die die Akupunkturpunktsuche unterstützt und wahlweise abschaltbar ist.

Fig. 1

zu Leiterplatte 7

21

18

17

16

10

13

15

14

19

20

12

Fig. 2

Europäisches
Patentamt

# EUROPÄISCHER RECHERCHENBERICHT

Nummer der Anmeldung

EP    92 81 0019

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-3 706 309 (TOFTNESS)<br>* Zusammenfassung; Abbildungen *<br>--- | 1 | A61H39/00 |
| A | FR-A-2 636 525 (FEBER ET AL.)<br>* Zusammenfassung; Abbildungen *<br>* Seite 1, Zeile 19 - Zeile 30 *<br>* Seite 2, Zeile 10 - Zeile 23 *<br>--- | 1 | |
| A,D | FR-A-2 419 057 (ASCHOFF ET AL.)<br>* Seite 2, Zeile 33 - Seite 3, Zeile 6;<br>Abbildungen *<br>* Seite 15, Zeile 10 - Zeile 38 *<br>--- | 1 | |
| A,D | FR-A-2 514 257 (CESKOSLOVENSKA AKADEMIE VED)<br>* Seite 2, Zeile 3 - Zeile 27; Abbildungen *<br>--- | 1 | |
| A | EP-A-0 074 914 (SERIEL (ETUDES ET REALISATIONS ELECTRONIQUES))<br>* Zusammenfassung; Abbildungen *<br>--- | 1 | |
| P,X | DE-U-9 100 366 (JENOPTIK CARL ZEISS JENA GMBH)<br>* das ganze Dokument *<br><br>----- | 1-10 | RECHERCHIERTE SACHGEBIETE (Int. Cl.5)<br><br>A61H<br>A61B |

Der vorliegende Recherchenbericht wurde für alle Patentansprüche erstellt

| Recherchenort | Abschlußdatum der Recherche | Prüfer |
|---|---|---|
| DEN HAAG | 13 APRIL 1992 | ZEINSTRA H. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes
    Dokument

EPO FORM 1503 03.82 (P0403)